# EUROPEAN PATENT APPLICATION

(11) **EP 4 535 068 A1**
(43) Date of publication of application: **09.04.2025**
(21) Application number: 23816122.8
(22) Date of filing: 31.05.2023
(51) Int. Cl.: G02C 7/04

(54) **SOFT CONTACT LENS HAVING COATING LAYER**

(30) Priority: 02.06.2022 JP 2022090437
(71) Applicant: NOF Corporation, Shibuya-ku Tokyo 150-6019 (JP)
(72) Inventor: GOTANDA Ryuya, Kawasaki-shi, Kanagawa 210-0865 (JP); IWAKIRI Norio, Kawasaki-shi, Kanagawa 210-0865 (JP)
(74) Representative: Hoffmann Eitle
(86) International application number: PCT/JP2023/020341
(87) International publication number: WO 2023/234365

(57) **Abstract**

Provided is a soft contact lens including a hydrogel having a glycidyl group, a first coating layer having a carboxyl group and/or an amino group, and a second coating layer having a glycidyl group, in which two coating layers are formed on a surface, and the surface has sufficient hydrophilicity and lubricity even in a case where the soft contact lens is worn continuously for a long period of time.

## Description

### TECHNICAL FIELD

The present invention relates to a soft contact lens including a coating layer excellent in surface hydrophilicity and lubricity, and a method of manufacturing the lens.

### BACKGROUND ART

It has been reported that wearers of soft contact lenses have rapidly increased in number due to ease and convenience of use of the soft contact lenses, and because of the ease and the convenience, a time of wearing the soft contact lenses is increasing particularly in younger populations (Non-Patent Literature 1). A problem caused by the increase in the time of wearing the soft contact lenses is a shortage of oxygen in the cornea of the wearer due to wearing the soft contact lens, and a feeling of discomfort caused by the wearing.

Since a silicone hydrogel soft contact lens can allow sufficient oxygen to permeate into the cornea, it is possible to minimize an adverse effect on the health of the cornea. However, due to hydrophobicity thereof, a dry sensation, a feeling of discomfort, or the like is given to the wearer, and thus hydrophilization of a surface of the contact lens is essential.

As a method for the hydrophilization, Patent Literatures 1 and 2 disclose "a technique for imparting and enhancing hydrophilicity to a surface of a soft contact lens and improving wearing comfort by blending polyethylene glycol, a cellulosic polymer, or the like in a treatment liquid for a soft contact lens".

Patent Literature 3 discloses "a technique of improving wearing comfort by adding a copolymer of a phosphorylcholine group-containing monomer and butyl methacrylate to a treatment liquid for a soft contact lens, the copolymer having a phospholipid-like structure derived from a biomembrane and being known to have very high hydrophilicity and moisturizing effect".

In the methods, although excellent hydrophilicity can be imparted to the soft contact lens, since adsorption is achieved on the surface of the contact lens only by physical interaction, there is a concern that the hydrophilicity becomes insufficient when the contact lens is worn for a whole day.

Patent Literature 4 discloses "a technique for performing a plasma treatment on a soft contact lens".

Patent Literature 5 discloses "a technique for hydrophilizing a surface of a soft contact lens by chemically bonding a soft contact lens having a reactive group and a hydrophilic polymer having a reactive group".

In the methods, by performing a treatment on the soft contact lens per se, sufficient hydrophilicity can be imparted to the surface of the soft contact lens even when the soft contact lens is worn for a whole day.

### PRIOR ART DOCUMENTS

### NON-PATENT LITERATURE

Non Patent Literature 1: Walline J. J., 2013, Long-term Contact Lens Wear of Children and Teens, Eye & Contact Lens, 39, 283-289.

### PATENT LITERATURE

Patent Literature 1: WO2009/032122
Patent Literature 2: WO2012/098653
Patent Literature 3: US2009/0100801
Patent Literature 4: WO2010/092686
Patent Literature 5: WO2001/074932

### SUMMARY OF INVENTION

### OBJECT TO BE ACHIEVED BY THE INVENTION

In the method disclosed in Patent Literature 4, since a surface treatment step is complicated, highly controlled manufacturing equipment is required, which is also economically disadvantageous. In the method disclosed in Patent Literature 5, an evaluation on durability of a coating is not performed, and there is a concern that hydrophilicity becomes insufficient in a case where the soft contact lens is worn continuously for a long period of time.

An object of the present invention is to provide a soft contact lens in which two coating layers are formed on a surface and which has sufficient hydrophilicity and lubricity on the surface even in a case where the soft contact lens is worn continuously for a long period of time.

### MEAND FOR ACHIEVING THE OBJECT

As a result of intensive studies, the present inventors have found that a soft contact lens including a hydrogel having a glycidyl group, a first coating layer having a carboxyl group and/or an amino group, and a second coating layer having a glycidyl group can solve the above problem, and have completed the present invention.

That is, the present invention is as follows.
1. A soft contact lens including the following (1), (2), and (3):
   (1) a hydrogel (P1) having a glycidyl group and containing a glycidyl (meth)acrylate constituent unit;
   (2) a first coating layer formed on a surface of the hydrogel and having a carboxyl group and/or an amino group, the first coating layer including, a first polymer (P2) containing a constituent unit derived from a carboxyl group-containing monomer and/or a constituent unit derived from an amino group-containing monomer, and/or polysaccharides having a carboxyl group and/or an amino group; and
   (3) a second coating layer formed on a surface of the first coating layer and having a glycidyl group, the second coating layer including a second polymer (P3) containing a constituent unit derived from glycidyl (meth)acrylate, and a constituent unit derived from 2-(meth)acryloyloxyethyl phosphorylcholine, polyethylene glycol (meth)acrylate, and/or methoxypolyethylene glycol methacrylate, in the soft contact lens,
   the glycidyl group of the hydrogel is bonded to the carboxyl group and/or the amino group of the first coating layer via a covalent bond, and the carboxyl group and/or the amino group of the first coating layer is bonded to the glycidyl group of the second coating layer via a covalent bond.
2. The soft contact lens according to 1, in which
   the first coating layer includes a first polymer (P2) containing a constituent unit derived from (meth)acrylic acid and/or a constituent unit derived from 2-aminoethyl (meth)acrylate.
3. The soft contact lens according to 1, in which
   the second coating layer includes a second polymer (P3) containing a constituent unit derived from 2-methacryloyloxyethyl phosphorylcholine.
4. A method of manufacturing the soft contact lens according to any of 1 to 3, the method including the following steps:
   (1) a step of immersing the hydrogel (P1) in a solution in which the first polymer (P2) and/or the polysaccharides having a carboxyl group and/or an amino group are dissolved; and
   (2) a step of immersing the hydrogel having the first coating layer formed thereon after the immersion in (1) in a solution in which the second polymer (P3) is dissolved.

### EFFECTS OF INVENTION

The soft contact lens according to the present invention has sufficient hydrophilicity and lubricity on the surface even in a case where the soft contact lens is worn continuously for a long period of time.

### EMBODIMENTS FOR CARRYING OUT THE INVENTION

### (Soft Contact Lens and Method of Manufacturing the Lens)

The present invention relates to a soft contact lens including the following (1), (2), and (3), and a method of manufacturing the lens.
(1) A hydrogel (P1) having a glycidyl group and containing a glycidyl (meth)acrylate constituent unit;
(2) a first coating layer formed on a surface of the hydrogel and having a carboxyl group and/or an amino group, the first coating layer including, a first polymer (P2) containing a constituent unit derived from a carboxyl group-containing monomer and/or a constituent unit derived from an amino group-containing monomer, and/or polysaccharides having a carboxyl group and/or an amino group; and
(3) a second coating layer formed on a surface of the first coating layer and having a glycidyl group, the second coating layer including a second polymer (P3) containing a constituent unit derived from glycidyl (meth)acrylate, and a constituent unit derived from 2-(meth)acryloyloxyethyl phosphorylcholine, polyethylene glycol (meth)acrylate, and/or methoxypolyethylene glycol methacrylate, in the soft contact lens,
the glycidyl group of the hydrogel is bonded to the carboxyl group and/or the amino group of the first coating layer via a covalent bond, and the carboxyl group and/or the amino group of the first coating layer is bonded to the glycidyl group of the second coating layer via a covalent bond.

In the present description, in a case where a preferable numerical range (for example, a concentration range) is described in stages, the respective lower limit values and upper limit values can be independently combined. For example, in a description of "preferably 10 or more, more preferably 20 or more, and preferably 100 or less, more preferably 90 or less", a "preferred lower limit value: 10" and a "more preferred upper limit value: 90 can be combined as "10 or more and 90 or less". In addition, for example, a description of "preferably 10 to 100, more preferably 20 to 90" may also be combined as "10 to 90" in the same manner.

In the present description, "(meth)acrylate" means "acrylate or methacrylate", and the same applies to other similar terms.

### <Regarding Hydrogel>

The hydrogel of the present invention is obtained by preparing a monomer composition for hydrogel in which glycidyl (meth)acrylate is mixed with one or more other monomers, polymerizing the monomer composition, and further hydrating the obtained polymer for hydrogel.

The hydrogel of the present invention contains constituent units derived from each monomer, which are based on each monomer or derived from each monomer.

Examples of the other monomers include a hydrophilic monomer, a hydrophobic monomer, a silicone-containing monomer, a crosslinking agent, and a UV-absorbing vinyl monomer.

A content of the glycidyl (meth)acrylate is, for example, generally 0.1 mass% to 20 mass%, and preferably 0.5 mass% to 10 mass% with respect to 100 mass% of the monomer composition constituting the hydrogel.

Examples of the hydrophilic monomer include hydroxy group-containing (meth)acrylates such as 2-hydroxyethyl (meth)acrylate, 2-hydroxypropyl (meth)acrylate, 2-hydroxybutyl (meth)acrylate, 4-hydroxybutyl (meth)acrylate, and glycerol (meth)acrylate; ionic group-containing monomers such as styrenesulfonic acid, (meth)acryloyloxyphosphonic acid, and 2-hydroxy-3-(meth)acryloyloxypropyltrimethylammonium chloride; nitrogen-containing monomers such as (meth)acrylamide, aminoethyl (meth)acrylate, N,N-dimethyl (meth)acrylamide, N,N-dimethylaminoethyl (meth)acrylate, N,N-dimethylaminopropyl (meth)acrylamide, N-acryloylmorpholine, 2-methacryloyloxyethyl phosphorylcholine, N-vinylpyrrolidone, N-vinylacetamide, and N-methyl-N-vinylacetamide; polyethylene glycol (meth) acrylate; and glycidyl (meth)acrylate.

As the hydrophilic monomer, one or two or more of the above may be used. A content of the hydrophilic monomer is generally 10 mass% to 99.9 mass%, preferably 20 mass% to 99 mass%, and more preferably 30 mass% to 98 mass% with respect to 100 mass% of the monomer composition constituting the hydrogel.

Examples of the hydrophobic monomer include linear or branched alkyl (meth) acrylates such as methyl (meth)acrylate, ethyl (meth)acrylate, butyl (meth)acrylate, 2-ethylhexyl (meth)acrylate, lauryl (meth)acrylate, and stearyl (meth)acrylate; cyclic alkyl (meth)acrylates such as cyclohexyl (meth)acrylate; aromatic (meth)acrylates such as benzyl (meth)acrylate and phenoxy ethyl (meth)acrylate; hydrophobic polyalkylene glycol (meth)acrylates such as polypropylene glycol (meth)acrylate; and styrene-based monomers such as styrene, methylstyrene, and chloromethylstyrene.

As the hydrophobic monomer, one or two or more of the above may be used. A content of the hydrophobic monomer is generally 1 mass% to 20 mass%, and preferably 5 mass% to 10 mass% with respect to 100 mass% of the monomer composition constituting the hydrogel.

Examples of the silicone-containing monomer include tris(trimethylsiloxy)silylpropyl methacrylate, 2-hydroxy-3-[3-[methylbis(trimethylsiloxy)silyl]proxy]propyl methacrylate, 2-(methacryloyloxy)ethyl=3-[tris(trimethylsiloxy)silyl]propyl=succinate, (meth)acryl polydimethylsiloxane, and methyldi(trimethylsiloxy)silyl propyl glycerol methacrylate.

As the silicone-containing monomer, one or two or more of the above may be used. A content of the silicone-containing monomer is generally 5 mass% to 90 mass%, preferably 15 mass% to 80 mass%, and more preferably 30 mass% to 70 mass% with respect to 100 mass% of the monomer composition constituting the hydrogel.

Examples of the crosslinking agent (monomer) include tetraethylene glycol di(meth)acrylate, triethylene glycol di(meth)acrylate, diethylene glycol di(meth)acrylate, ethylene glycol di(meth)acrylate, tetraethylene glycol divinyl ether, triethylene glycol divinyl ether, diethylene glycol divinyl ether, ethylene glycol divinyl ether, trimethylolpropane trimethacrylate, pentaerythritol tetramethacrylate, bisphenol A dimethacrylate, vinyl methacrylate, ethylenediamine di-(meth)acrylamide, glycerol dimethacrylate, triallyl isocyanurate, triallyl cyanurate, allyl (meth)acrylate, N-allyl-(meth)acrylamide, 1,3-bis(methacrylamidopropyl)-1,1,3,3-tetrakis (trimethylsiloxy) disiloxane, N,N'-methylene bis(meth)acrylamide, N,N'-ethylene bis(meth)acrylamide, and N,N'-dihydroxyethylene bis(meth)acrylamide.

As the crosslinking agent, one or two or more of those may be used, and tetraethylene glycol di(meth)acrylate, ethylene glycol di(meth)acrylate, glycerol dimethacrylate, or triethylene glycol divinyl ether is preferable.

A content of the crosslinking agent is generally 0.01 mass% to 10 mass%, preferably 0.1 mass% to 10 mass%, and more preferably 0.2 mass% to 5 mass% with respect to 100 mass% of the monomer composition constituting the hydrogel.

As a monomer other than those described above, a UV-absorbing vinyl monomer may be blended into the monomer composition for hydrogel. Examples of the UV-absorbing vinyl monomer include 2-(2-hydroxy-5-vinylphenyl)-2H-benzotriazole, 2-(2-hydroxy-5-acrylyloxy)phenyl)-2H-benzotriazole, 2-(2-hydroxy-3-methacrylamide methyl-5-tert-octylphenyl) benzotriazole, 2-(2'-hydroxy-5'-methacrylamide phenyl)-5-chlorobenzotriazole, 2-(2'-hydroxy-5'-methacrylamide phenyl)-5-methoxybenzotriazole, 2-(2'-hydroxy-5'-methacryloxypropyl-3'-t-butyl-phenyl)-5-chlorobenzotriazole, 2-(2'-hydroxy-5'-methacryloxypropyl phenyl) benzotriazole, 2-hydroxy-5-methoxy-3-(5-(trifluoromethyl)-2H-benzo[d][1,2,3]triazole-2-yl) benzyl methacrylate, 2-hydroxy-5-methoxy-3-(5-methoxy-2H-benzo[d][1,2,3]triazole-2-yl) benzyl methacrylate, 3-(5-fluoro-2H-benzo[d][1,2,3]triazole-2-yl)-2-hydroxy-5-methoxybenzyl methacrylate, 3-(2H-benzo[d][1,2,3]-triazole-2-yl)-2-hydroxy-5-methoxybenzyl methacrylate, 3-(5-chloro-2H-benzo[d][1,2,3]triazole-2-yl)-2-hydroxy-5-methoxybenzyl methacrylate, 2-hydroxy-5-methoxy-3-(5-methyl-2H-benzo[d][1,2,3]triazole-2-yl) benzyl methacrylate, 2-hydroxy-5-methyl-3-(5-(trifluoromethyl)-2H-benzo[d][1,2,3]triazole-2-yl) benzyl methacrylate, 4-allyl-2-(5-chloro-2H-benzo[d][1,2,3]-triazole-2-yl)-6-methoxyphenol, 2-{2'-hydroxy-3'-tert-5'[3"-(4"-{vinylbenzyloxy)propoxy]phenyl}-5-methoxy-2H-benzotriazole, phenol, 2-(5-chloro-2H-benzotriazole-2-yl)-6-(1,1-dimethylethyl)-4-ethenyl, 2-(2'-hydroxy-5'-methacryloxyethylphenyl) benzotriazole (2-propenoic acid, 2-methyl-, 2-[3-(2H-benzotriazole-2-yl)-4-hydroxyphenyl] ethyl ester, Norbloc), 2-{2'-hydroxy-3'-tert-butyl-5'-[3'-methacryloyloxypropoxy]phenyl}-5-methoxy-2H-benzotriazole, 2-[2'-hydroxy-3'-tert-butyl-5'-(3'-acryloyloxypropoxy)phenyl]-5-trifluoromethyl-2H-benzotriazole, 2-(2'-hydroxy-5-methacrylamide phenyl)-5-methoxybenzotriazole, 2-(3-allyl-2-hydroxy-5-methylphenyl)-2H-benzotriazole, 2-(2-hydroxy-3-methyl-5-methylphenyl)-2H-benzotriazole, 2-3'-t-butyl-2'-hydroxy-5'-(3"-dimethylvinylsilylpropoxy)-2'-hydroxy-phenyl)-5-methoxybenzotriazole, 2-(2'-hydroxy-5'-methacryloylpropyl-3'-tert-butyl-phenyl)-5-methoxy-2H-benzotriazole, 2-(2'-hydroxy-5'-acryloylpropyl-3'-tert-butyl-phenyl)-5-methoxy-2H-benzotriazole, 2-methylacrylic acid 3-[3-tert-butyl-5-(5-chlorobenzotriazole-2-yl)-4-hydroxyphenyl]-propyl ester (CAS#96478-15-8), and 2-(3-(tert-butyl)-4-hydroxy-5-(5-methoxy-2H-benzo[d][1,2,3]triazole-2-yl)phenoxy) ethyl methacrylate; phenol, and 2-(5-chloro-2H-benzotriazole-2-yl)-6-methoxy-4-(2-propene-1-yl) (CAS#1260141-20-5); 2-[2-hydroxy-5-[3-(methacryloyloxy)propyl]-3-tert-butyl phenyl]-5-chloro-2H-benzotriazole; phenol, 2-(5-ethenyl-2H-benzotriazole-2-yl)-4-methyl-, homopolymer (9CI) (CAS#83063-87-0), and the like.

A content of the UV-absorbing vinyl monomer is generally 0.1 parts by mass to 30 parts by mass, preferably 0.2 parts by mass to 20 parts by mass, and more preferably 0.3 parts by mass to 10 parts by mass with respect to 100 parts by mass of the monomer composition constituting the hydrogel.

The monomer composition for hydrogel may contain a solvent as necessary. As the solvent, a solvent which does not react under polymerization conditions can be used, and examples thereof include water, tetrahydrofuran, tripropylene glycol methyl ether, dipropylene glycol methyl ether, ethylene glycol n-butyl ether, acetone, methyl ethyl ketone, diethylene glycol n-butyl ether, diethylene glycol methyl ether, ethylene glycol phenyl ether, propylene glycol methyl ether, propylene glycol methyl ether acetate, dipropylene glycol methyl ether acetate, propylene glycol n-propyl ether, dipropylene glycol n-propyl ether, tripropylene glycol n-butyl ether, propylene glycol n-butyl ether, dipropylene glycol n-butyl ether, tripropylene glycol n-butyl ether, propylene glycol phenyl ether dipropylene glycol dimethyl ether, polyethylene glycol, polypropylene glycol, ethyl acetate, butyl acetate, amyl acetate, methyl lactate, ethyl lactate, isopropyl lactate, methylene chloride, chloroform, ethanol, 1-propanol, 2-propanol, 2-butanol, menthol, 1-hexanol, cyclohexanol, cyclopentanol, exo-norborneol, 2-pentanol, 3-pentanol, 2-hexanol, 3-hexanol, 3-methyl-2-butanol, 2-heptanol, 2-octanol, 2-nonanol, 2-decanol, 3-octanol, norborneol, tert-butanol, tert-amyl alcohol, 2-methyl-2-pentanol, 2,3-dimethyl-2-butanol, 3-methyl-3-pentanol, 1-methylcyclohexanol, 2-methyl-2-hexanol, 3,7-dimethyl-3-octanol, 1-chloro-2-methyl-2-propanol, 2-methyl-2-heptanol, 2-methyl-2-octanol, 2-2-methyl-2-nonanol, 2-methyl-2-decanol, 3-methyl-3-hexanol, 3-methyl-3-heptanol, 4-methyl-4-heptanol, 3-methyl-3-octanol, 4-methyl-4-octanol, 3-methyl-3-nonanol, 4-methyl-4-nonanol, 3-methyl-3-octanol, 3-ethyl-3-hexanol, 3-methyl-3-heptanol, 4-ethyl-4-heptanol, 4-propyl-4-heptanol, 4-isopropyl-4-heptanol, 2,4-dimethyl-2-pentanol, 1-methylcyclopentanol, 1-ethylcyclopentanol, 1-ethylcyclopentanol, 3-hydroxy-3-methyl-1-butene, 4-hydroxy-4-methyl-1-cyclopentanol, 2-phenyl-2-propanol, 2-methoxy-2-methyl-2-propanol 2,3,4-trimethyl-3-pentanol, 3,7-dimethyl-3-octanol, 2-phenyl-2-butanol, 2-methyl-1-phenyl-2-propanol, 3-ethyl-3-pentanol, 1-ethoxy-2-propanol, 1-methyl-2-propanol, 1-methyl-2-pyrrolidone, N,N-dimethylpropionamide, dimethyl sulfoxide, dimethylformamide, dimethylacetamide, and N-methylpyrrolidinone, and one kind or two or more kinds thereof can be used.

The polymerization of the monomer composition for hydrogel may be performed by a conventionally known method. For example, the polymerization can be performed using a known polymerization initiator such as a thermal polymerization initiator or a photopolymerization initiator.

The thermal polymerization initiator is known to those skilled in the art, and examples thereof include azo-based polymerization initiators such as 2,2'-azobisisobutyronitrile, dimethyl 2,2-azobis(2-methylpropionate) 2,2'-azobis[2-(2-imidazoline-2-yl)propane] dihydrochloride, 2,2'-azobis(2,4-dimethylvaleronitrile), 2,2'-azobis[2-(2-imidazoline-2-yl) propane] disulfate dihydrate, 2,2'-azobis(2-methylpropionamidine) dihydrochloride, 2,2'-azobis[N-(2-carboxyethyl)-2-methylpropionamidine] dihydrate, 2,2'-azobis[2-(2-imidazoline-2-yl) propane], 2,2'-azobis(1-imino-1-pyrrolizino-2-methyl propane) dihydrochloride, 2,2'-azobis[2-methyl-N-{1,1-bis(hydroxymethyl)-2-hydroxyethyl} propionic amide], 2,2'-azobis[2-methyl-N-(2-hydroxyethyl) propionic amide], 2,2'-azobis(2-methylpropionamidine) dihydrochloride, and 2,2'-azobis[2-methyl-N-(2-hydroxyethyl) propionic amide]; and peroxide polymerization initiators such as benzoyl peroxide, t-butyl hydroperoxide, cumene hydroperoxide, lauroyl peroxide, t-butylperoxy hexanoate, and 3,5,5-trimethylhexanoyl peroxide, and one kind or two or more kinds thereof can be used.

Azo-based polymerization initiators are preferable from the viewpoint of safety and availability, and 2,2'-azobisisobutyronitrile, dimethyl 2,2-azobis(2-methylpropionate), and 2,2'-azobis(2,4-dimethylvaleronitrile) are particularly preferable from the viewpoint of reactivity.

Examples of the photopolymerization initiator include acetophenone, benzophenone, 2-benzoylbenzoic acid, 4-benzoylbenzoic acid, methyl 2-benzoylbenzoate, benzoin methyl ether, benzoin ethyl ether, benzoin isobutyl ether, diethoxyacetophenone, 2-isonitrosopropiophenone, 2-ethylanthraquinone, 2,4-diethylthioxanthen-9-one, phenyl bis(2,4,6-trimethylbenzoyl) phosphine oxide, bis-(2,6-dichlorobenzoyl)-4-N-propylphenylphosphine oxide, and bis-(2,6-dichlorobenzoyl)-4-N-butylphenylphosphine oxide, which are of Darocur (registered trademark) types and Irgacur (registered trademark) types.

Darocur (registered trademark) 1173, Darocur (registered trademark) 2959, and Irgacur (registered trademark) 819 are preferable. For example, a reactive photoinitiator that can be incorporated into a macromer or can be used as a special monomer is also preferred.

The polymer for hydrogel can be obtained by a method known to those skilled in the art, and is manufactured, for example, by using a mold (gold mold) having a hydrophobic surface made of polypropylene or the like, filling the mold with the monomer composition for hydrogel, and polymerizing the monomer composition.

Polymerization reaction may be performed in the air, and may be performed in an inert gas atmosphere such as nitrogen or argon for the purpose of improving a polymerization rate. In a case where the polymerization is performed in an inert gas atmosphere, a pressure in a polymerization system is preferably 1 kgf/cm² or less.

The polymer for hydrogel obtained as described above can be peeled off from the mold by a known method and taken out in a dry state. In addition, the polymer for hydrogel may be immersed in a solvent (for example, water, methanol, ethanol, 1-propanol, 2-propanol, and a mixed solution thereof) together with the mold to swell and peel off a contact lens base material.

Further, a residue, a retentate, a byproduct, and the like of each component can be removed by washing by means of repeatedly immersing the polymer for hydrogel in the solvent. The washing can be performed by, for example, immersing the polymer for hydrogel in the above alcohol or an alcohol aqueous solution having a concentration of 30 mass% or more at 10°C to 40°C.

After the washing, washing may be further performed by immersing the polymer for hydrogel in an alcohol aqueous solution having an alcohol concentration of 20 mass% to 50 mass% for 10 minutes to 5 hours.

The hydrogel of the present invention can be obtained by immersing the polymer for hydrogel in water for 10 minutes to 5 hours after the washing in alcohol.

Before the washing in water, a washing step of immersing the polymer for hydrogel in an alcohol aqueous solution having an alcohol concentration of 20 mass% to 50 mass% for 10 minutes to 5 hours may be added. Water is preferably pure water such as RO water, ion-exchanged water, and distilled water.

### <Regarding First Coating Layer>

The first polymer (P2) constituting (included in) the first coating layer is not particularly limited as long as the first polymer (P2) including a first polymer (P2) containing a constituent unit derived from a carboxyl group-containing monomer and/or a constituent unit derived from an amino group-containing monomer and/or polysaccharides having a carboxyl group and/or an amino group.

The constituent unit means a unit of a compound contained in a polymer based on each monomer or derived from each monomer.

Examples of the carboxyl group-containing monomer (carboxyl group-containing compound) include N,N-2-acrylamidoglycolic acid, β-methyl-acrylic acid (crotonic acid), β-acryloxypropionic acid, sorbic acid, angelic acid, cinnamic acid, 1-carboxy-4-phenyl-1, 3-butadiene, itaconic acid, citraconic acid, mesaconic acid, glutaconic acid, aconitic acid, maleic acid, fumaric acid, (meth)acrylic acid, 2-(meth)acryloyloxyethyl succinic acid, 2-(meth)acryloyloxyethyl hexahydrophthalic acid, 2-(meth)acryloyloxyethyl-phthalic acid, and vinylbenzoic acid.

In addition, the polymer having a carboxyl group includes, as main components, poly (meth)acrylic acid, alginic acid, xanthan gum, gellan gum, gum arabic, pectin, carrageenan, karaya gum, succinoglycan, hyaluronic acid, chondroitin sulfate, polyethylene glycol (PEG) having one carboxyl group at a terminal (for example, HO-PEG-COOH, H3CO-PEG-COOH), PEG (HOOC-PEG-COOH) having carboxyl groups at both terminals, multi-arm PEG having one or more carboxyl groups, PEG dendrimer, and the like. The (meth)acrylic acid is preferable, and the methacrylic acid is more preferable.

Examples of the amino group-containing monomer (amino group-containing compound) include aminoethyl methacrylate (2-aminoethyl methacrylate), dimethylaminoethyl (meth)acrylate, and N,N-dimethylaminopropyl (meth)acrylamide. Aminoethyl methacrylate (2-aminoethyl methacrylate) is preferable.

The first polymer (P2) may be a copolymer with another monomer other than the compounds described above, and examples of the another monomer include (meth)acrylic esters such as methyl (meth)acrylate, ethyl (meth)acrylate, butyl (meth)acrylate, 2-ethylhexyl (meth)acrylate, stearyl (meth)acrylate, perfluoroalkylethyl (meth)acrylate, phenyl (meth)acrylate, 2-hydroxyethyl (meth)acrylate, 2-amino ethyl (meth)acrylate and salts thereof, methoxy polyethylene glycol (meth)acrylate, and monoesters of (meth)acrylate and polyethylene glycol; unsaturated nitriles such as (meth)acrylonitrile; unsaturated amides such as (meth)acrylamide, N-methylol (meth)acrylamide, and N-(2-hydroxyethyl) (meth)acrylamide; vinyl esters such as vinyl acetate and vinyl propionate; vinyl ethers such as methyl vinyl ether and ethyl vinyl ether; α-olefins such as ethylene and propylene; halogen-containing α,β-unsaturated monomers such as vinyl chloride, vinylidene chloride, and vinyl fluoride; and α,β-unsaturated aromatic monomers such as styrene, α-methylstyrene, and sodium styrene sulfonate. The butyl (meth)acrylate is preferable, and the butyl methacrylate is more preferable.

An amount of the constituent unit derived from the carboxyl group-containing monomer and/or the constituent unit derived from the amino group-containing monomer contained in the above first polymer (P2) is preferably 10 mol% to 95 mol%, more preferably 20 mol% to 90 mol%, and further preferably 25 mol% to 80 mol% with respect to 100 mol% of all constituent units.

A weight average molecular weight of the above first polymer (P2) is preferably 5,000 to 1,000,000, and more preferably 100,000 to 800,000.

As the above first polymer (P2), a commercially available polymer may be used.

Examples of the polysaccharides having a carboxyl group and/or an amino group include alginic acid, xanthan gum, gellan gum, gum arabic, pectin, carrageenan, karaya gum, succinoglycan, hyaluronic acid, and chondroitin sulfate.

### <Regarding Second Coating Layer>

The second polymer (P3) constituting (included in) the second coating layer is not particularly limited as long as the second polymer (P3) contains a constituent unit derived from glycidyl (meth)acrylate and a constituent unit derived from 2-(meth)acryloyloxyethyl phosphorylcholine, polyethylene glycol (meth)acrylate, and/or methoxypolyethylene glycol methacrylate.

The constituent unit means a unit of a compound contained in a polymer based on each monomer or derived from each monomer.

The second polymer (P3) may be a copolymer with another monomer other than the compounds of glycidyl (meth)acrylate, as well as 2-(meth)acryloyloxyethyl phosphorylcholine, polyethylene glycol (meth)acrylate, and/or methoxypolyethylene glycol methacrylate, and examples of the another monomer include hydroxy group-containing (meth)acrylates such as 2-hydroxyethyl (meth)acrylate, 2-hydroxypropyl (meth)acrylate, 2-hydroxybutyl (meth)acrylate, 4-hydroxybutyl (meth)acrylate, and glycerol (meth)acrylate; ionic group-containing monomers such as styrenesulfonic acid, (meth)acryloyloxyphosphonic acid, and 2-hydroxy-3-(meth)acryloyloxypropyltrimethylammonium chloride; and nitrogen-containing monomers such as (meth)acrylamide, N,N-dimethyl (meth)acrylamide, N-acryloylmorpholine, N-vinylpyrrolidone, N-vinylacetamide, and N-methyl-N-vinylacetamide.

As a hydrophilic monomer other than a monomer having a glycidyl group, one kind or two or more kinds of those can be used, and 2-methacryloyloxyethyl phosphorylcholine, N-vinylpyrrolidone, and polyethylene glycol (meth)acrylate are preferable, and - methacryloyloxyethyl phosphorylcholine (MPC) is more preferable.

An amount of the constituent unit derived from glycidyl (meth)acrylate contained in the above second polymer is preferably 5 mol% to 95 mol%, more preferably 7 mol% to 90 mol%, and further preferably 10 mol% to 80 mol% with respect to 100 mol% of all constituent units.

A weight average molecular weight of the above second polymer is preferably 5,000 to 1,000,000, and more preferably 100,000 to 800,000.

### <Polymerization Reaction>

The polymerization reaction for obtaining the above first polymer (first coating layer) and second polymer (second coating layer) can be performed by a known method such as radical polymerization, for example, bulk polymerization, suspension polymerization, emulsion polymerization, and solution polymerization in the presence of a radical polymerization initiator by performing substitution with or in an atmosphere of an inert gas such as nitrogen, carbon dioxide, argon, or helium. From the viewpoint of purification and the like, solution polymerization is preferable. Purification of the polymer can be performed by a general purification method such as reprecipitation, dialysis, or ultrafiltration.

Examples of the radical polymerization initiator include azo-based radical polymerization initiators, organic peroxides, and persulfates.

Examples of the azo-based radical polymerization initiator include 2,2'-azobis(2-methylpropionamidine) dihydrochloride, 2,2-azobis(2-diaminopropyl) dihydrochloride, 2,2-azobis(2-(5-methyl-2-imidazoline-2-yl) propane) dihydrochloride, 4,4-azobis(4-cyanovaleric acid), 2,2-azobisisobutyramide dihydrate, 2,2-azobis(2,4-dimethylvaleronitrile), and 2,2-azobisisobutyronitrile (AIBN).

Examples of the organic peroxide include t-butyl peroxyneodecanoate (Perbutyl (registered trademark) ND), benzoyl peroxide, diisopropyl peroxydicarbonate, t-butylperoxy-2-ethylhexanoate, t-butylperoxy pivalate, t-butylperoxy diisobutyrate, lauroyl peroxide, and succinic acid peroxide (= succinyl peroxide).

Examples of the persulfate include ammonium persulfate, potassium persulfate, and sodium persulfate.

These radical polymerization initiators may be used alone or in a mixture of two or more kinds thereof. A usage amount of the polymerization initiator is generally 0.001 parts by mass to 10 parts by mass, and preferably 0.01 parts by mass to 5.0 parts by mass with respect to 100 parts by mass of a monomer composition of the hydrophilic polymer.

The polymerization reaction can be performed in the presence of a solvent, and as the solvent, a solvent that dissolves a monomer composition and does not react can be used. Examples of the solvent include water; alcohol-based solvents such as methanol, ethanol, n-propanol, and isopropanol; ketone-based solvents such as acetone, methyl ethyl ketone, and diethyl ketone; ester-based solvents such as ethyl acetate; linear or cyclic ether-based solvents such as ethyl cellosolve, tetrahydrofuran, and N-methylpyrrolidone; and nitrogen-containing solvents such as acetonitrile and nitromethane. Preferred examples thereof include water or a mixed solvent thereof.

### <Method of Manufacturing Soft Contact Lens>

The method of manufacturing the soft contact lens of the present invention is not particularly limited as long as the method includes a step of bonding the hydrogel and the first polymer to each other via a covalent bond and further bonding the first polymer and the second polymer to each other via a covalent bond. For example, the following steps can be exemplified.
(1) A step of immersing the hydrogel (P1) in a solution in which the first polymer (P2) and/or the polysaccharides having a carboxyl group and/or an amino group are dissolved.
(2) A step of immersing the hydrogel having the first coating layer formed thereon after the immersion in (1) in a solution in which the second polymer (P3) is dissolved.

### <Reaction between Hydrogel and First Coating Layer>

The hydrogel and the first coating layer (first polymer) are bonded to each other via a covalent bond by causing a glycidyl group in the hydrogel to react with a carboxyl group and/or an amino group in the first polymer.

The above reaction is performed by immersing the hydrogel in a solution in which the first polymer is dissolved, at a temperature of about 20°C to about 90°C for about 0.2 hours to about 24 hours, preferably about 0.5 hours to about 12 hours, and further preferably about 1 hour to 6 hours.

The solvent in which the first polymer is dissolved is not particularly limited as long as the solvent is a solvent that dissolves the first polymer and does not react, and is preferably water, methanol, ethanol, 1-propanol, 2-propanol, or a mixed solvent thereof.

The above reaction may be performed together with a step of washing the polymer for hydrogel. For example, the above reaction is performed by immersing the hydrogel in a washing solution in which the first polymer is dissolved and washing the hydrogel at a temperature of about 20°C to about 90°C for about 0.2 hours to about 24 hours, preferably about 0.5 hours to about 12 hours, and further preferably about 1 hour to 6 hours.

### <Reaction between First Coating Layer and Second Coating Layer>

In the reaction between the first coating layer and the second coating layer, the carboxyl group and/or the amino group in the first polymer and the glycidyl group in the second polymer are caused to react with each other to be bonded through a covalent bond.

The above reaction is performed by immersing the hydrogel obtained by causing the first polymer to react in a solution in which the second polymer is dissolved, at a temperature of about 20°C to about 125°C for about 0.2 hours to about 24 hours, preferably about 0.5 hours to about 12 hours, and further preferably about 1 hour to 6 hours.

The solvent in which the second polymer is dissolved is not particularly limited as long as the solvent is a solvent that dissolves the second polymer and does not react, and is preferably water, methanol, ethanol, 1-propanol, 2-propanol, or a mixed solvent thereof.

The above reaction may be performed together with a step of washing the hydrogel caused to react with the first polymer. For example, the above reaction is performed by immersing the hydrogel caused to react with the first polymer in a washing solution in which the second polymer is dissolved and washing the hydrogel at a temperature of about 20°C to about 90°C for about 0.2 hours to about 24 hours, preferably about 0.5 hours to about 12 hours, and further preferably about 1 hour to 6 hours.

The above reaction may be performed together with a sterilization step of the hydrogel caused to react with the first polymer. For example, the hydrogel caused to react with the first polymer is immersed in a lens package or a sealable vial together with a physiological saline solution in which the second polymer is dissolved. Thereafter, the above reaction is performed by performing an autoclave treatment at a temperature of about 118°C to about 125°C for about 20 minutes to 90 minutes.

### <Configuration Example of Soft Contact Lens of the Present Invention>

Configuration examples of the soft contact lens of the present invention include, but are not particularly limited to, the following combinations.
(1) The hydrogel contains a constituent unit derived from glycidyl methacrylate, the first coating layer contains a constituent unit derived from methacrylic acid, and the second coating layer contains a constituent unit derived from glycidyl methacrylate and a constituent unit derived from 2-(meth)acryloyloxyethyl phosphorylcholine.
(2) The hydrogel contains a constituent unit derived from glycidyl methacrylate, the first coating layer contains a constituent unit derived from 2-aminoethyl methacrylate, and the second coating layer contains a constituent unit derived from glycidyl methacrylate and a constituent unit derived from methoxypolyethylene glycol methacrylate.
(3) The hydrogel contains a constituent unit derived from glycidyl methacrylate, the first coating layer contains a polyacrylic acid (having a preferable weight average molecular weight of 5,000 to 1,000,000), and the second coating layer contains a constituent unit derived from glycidyl methacrylate and a constituent unit derived from 2-(meth)acryloyloxyethyl phosphorylcholine.

The present invention will be described in detail below according to Examples, but the present invention is not limited to these Examples.

Components used in Synthesis Examples, Examples, and Comparative Examples are shown below.
GdMA: glycidyl methacrylate
HEMA: 2-hydroxyethyl methacrylate
HBMA: 2-hydroxybutyl methacrylate
NVP: N-vinylpyrrolidone
DMAA: N,N-dimethylacrylamide
MMA: methyl methacrylate
ETS: 2-(methacryloyloxy) ethyl=3-[tris(trimethylsiloxy) silyl] propyl=succinate
SiGMA: methyl di(trimethylsiloxy) silyl propyl glycerol methacrylate
MCR-M11: polydimethylsiloxane one-terminal methacrylate having a molecular weight of about 1,000
TEGDMA: tetraethylene glycol dimethacrylate
EtOH: ethanol
NPA: 1-propanol (normal propanol)
AIBN: 2,2'-azobis(isobutyronitrile)
I-819: Irgacur (registered trademark) 819 (phenylbis(2,4,6-trimethylbenzoyl) phosphine oxide)
MAA: methacrylic acid
PAA: polyacrylic acid
AEMA: 2-aminoethyl methacrylate
MPC: 2-methacryloyloxyethyl phosphorylcholine
PME-1000: methoxypolyethylene glycol methacrylate having a molecular weight of about 1,000
BMA: butyl methacrylate
PB-ND: t-butyl peroxyneodecanoate
V-50: 2,2'-azobis(2-methylpropionamidine) dihydrochloride

### [Adjustment of Hydrogel]

### <Synthesis Example 1-1>

2.0 mass% of GdMA, 12.5 mass% of HBMA, 30.0 mass% of NVP, 5.0 mass% of MMA, 20.0 mass% of SiGMA, 30.0 mass% of MCR-M11, 0.50 mass% of TEGDMA, and 10.0 parts by mass of NPA were mixed. Subsequently, 0.5 parts by mass of AIBN was dissolved therein to obtain a composition for hydrogel.

The composition for hydrogel was dispensed into a mold and heated in an oven for polymerization set to 100°C for 2 hours to obtain a polymer for hydrogel.

The polymer is taken out from the mold, immersed in about 20 g of a solution of 2-propanol:ion-exchanged water = 1:1 for 4 hours, and then immersed in about 20 g of ion-exchanged water for 2 hours to obtain a hydrogel having a glycidyl group.

### <Synthesis Examples 1-2 and 1-3>

Hydrogels having a glycidyl group were adjusted in the same manner as in Synthesis Example 1-1 except that a blending ratio of each component in the composition and polymerization conditions were changed as shown in Table 1.

In Synthesis Example 1-2, a composition for hydrogel dispensed into the mold was placed in a UV-LED irradiator (wavelength of irradiation light: 405 nm), and subjected to UV irradiation at room temperature at an irradiation intensity of 1.5 mW/cm² for 30 minutes, thereby obtaining a polymer for hydrogel.

### [Adjustment of First Polymer Solution (First Coating Layer Solution)]

### <Synthesis Example 2-1>

A polymerization solution was obtained by dissolving 19.4 g of MAA and 3.6 g of BMA in 57.5 g of water and 57.5 g of EtOH. After a temperature of the polymerization solution was raised to 55°C under a nitrogen atmosphere, 0.12 g of V-50 was added to initiate polymerization. After 3 hours, the temperature thereof was raised to 75°C, and the mixture was further reacted for 2 hours, thereby obtaining a copolymer solution. After impurities were removed from the obtained polymer solution by dialysis purification, the solution was diluted with water such that a concentration of the first polymer became 5 mass%, thereby obtaining a first aqueous polymer solution.

A method of measuring a weight average molecular weight of the obtained copolymer is as follows. In addition, measurements were performed in the same manner also in Synthesis Examples 2-2 to 3-1 to 3-3.
1 mg of the copolymer was dissolved in 1 g of purified water.
Column: SB-802.5 HQ+SB-806MN HQ
Mobile phase: 20 mM of phosphate buffer (pH: 7.0)
Standard substance: polyethylene glycol/oxide
Measurement instrument: HLC-8320GPC (manufactured by TOSOH CORPORATION)
Method of calculating weight average molecular weight: molecular weight calculation program (EcoSEC Date Analysis)
Flow rate: 0.5 mL per minute
Injection amount: 100 µL
Column oven: 45°C
Measurement time: 70 minutes

### <Synthesis Example 2-2>

A polymerization solution was obtained by dissolving 23.2 g of AEMA and 2.8 g of BMA in 65.0 g of water and 65.0 g of EtOH. After a temperature of the polymerization solution was raised to 55°C under a nitrogen atmosphere, 0.13 g of V-50 was added to initiate polymerization. After 3 hours, the temperature thereof was raised to 75°C, and the mixture was further reacted for 2 hours, thereby obtaining a copolymer solution. After impurities were removed from the obtained polymer solution by dialysis purification, the solution was diluted with water such that a concentration of the first polymer became 5 mass%, thereby obtaining a first aqueous polymer solution.

### <Synthesis Example 2-3>

A commercially available polymer may be used as the first polymer, and the first polymer was diluted with water using a commercially available PAA (average molecular weight: 250,000) such that a concentration of the first polymer was 5 mass%, thereby obtaining a first polymer solution.

### [Adjustment of Second Polymer Solution (Second Coating Layer Solution)]

### <Synthesis Example 3-1>

2.8 g of GdMA and 23.6 g of MPC were dissolved in a mixed solvent of 52.8 g of water and 52.8 g of ethanol to obtain a polymerization solution. After a temperature of the polymerization solution was raised to 55°C under a nitrogen atmosphere, 0.17 g of PB-ND was added to initiate polymerization. After 3 hours, the temperature thereof was raised to 75°C, and the mixture was further reacted for 2 hours, thereby obtaining a copolymer solution. After impurities were removed from the obtained polymer solution by dialysis purification, the solution was diluted with water such that a concentration of the second polymer became 5 mass%, thereby obtaining a second aqueous polymer solution.

### <Synthesis Examples 3-2 and 3-3>

Second aqueous polymer solutions were obtained in the same manner as in Synthesis Example 3-1 except that an amount of each component in the polymerization solution and polymerization conditions were changed as shown in Table 2.

### [Evaluation]

### <Transparency>

Transparency of the hydrogel (before coating) and the contact lens (after coating) was visually evaluated.

### <Moisture Content>

Moisture contents of the hydrogel and the contact lens were measured in accordance with a method described in ISO18369-4.

### <Elongation at Break>

Elongations at break of the hydrogel and the contact lens were measured in accordance with JIS-K7127 using a breaking strength analyzer of BAS-3305(W) manufactured by YAMADEN co.,ltd. A 2N load cell was used. As a measurement sample, a hydrogel or a contact lens cut into a width of 2 mm was used, pulled at a rate of 1 mm/s at a clamp interval of 6 mm, and the elongation at break was evaluated from a position where the sample broke.

### <Oxygen Permeability Coefficient (Dk)>

An oxygen permeability coefficient of the contact lens was measured in accordance with the method described in ISO18369-4.

### <WBUT: Water Film Breaking Up Time>

For WBUTs of the hydrogel and the contact lens, the hydrogel and the contact lens were quietly taken out, and a time until a water film on a surface thereof was cut was measured with a stopwatch.

When the WBUT was 10 seconds or longer, it was determined that wettability was good.

### <Lubricity>

For comparison, lubricity at fingertips of polymacon (soft contact lens containing 2-hydroxyethyl methacrylate (HEMA) as a main component) and omafilcon A (soft contact lens containing 2-hydroxyethyl methacrylate and 2-methacryloyloxyethyl phosphorylcholine as main components) was evaluated by sensory evaluation.

The evaluation was performed on a 10-grade scale, and scores were given as follows when surface lubricity of polymacon was 2 and surface lubricity of omafilcon A was 8.
1: lubricity is lowest.
10: lubricity is highest.

When the score was 6 or more, it was determined that the lubricity was good.

### <Durability>

The contact lens was rinsed with a physiological saline solution, then placed on a palm of a hand opposite to the dominant hand with an inner side of the lens facing upward, and 1 mL of the physiological saline solution was dropped thereon. A pad of an index finger of the dominant hand was brought into contact with an inner side of the contact lens and lightly pressed, the lens was moved forward, backward, leftward, and rightward, and rubbed 30 times for washing. After the operation was repeated 14 times and 30 times, the lubricity and the wettability were evaluated, respectively.
A: wettability and lubricity were not reduced.
B: wettability or lubricity was reduced.
C: wettability and lubricity were reduced.

### <Example 1>

The hydrogel adjusted in Synthesis Example 1-1 was used as the hydrogel. The hydrogel was immersed in the first polymer solution adjusted in Synthesis Example 2-1, and heated at 75°C for 2 hours in the presence of triethylamine in an amount of catalyst, thereby bonding the hydrogel and the first polymer to each other via a covalent bond and forming a first coating layer on a surface of the hydrogel.

Next, the hydrogel having the first coating layer was immersed in the second polymer solution adjusted in Synthesis Example 3-1, and heated at 75°C for 2 hours in the presence of triethylamine in an amount of catalyst, thereby obtaining a contact lens in which the second polymer was bonded to the first polymer via a covalent bond and a hydrophilic coating layer was formed on the surface.

The obtained contact lens was sufficiently washed with water, then immersed in a physiological saline solution adjusted in accordance with ISO18369-3, and subjected to a sterilization treatment under a condition of 121°C and 20 minutes, and each evaluation was performed.

### <Examples 2 and 4>

Contact lenses having a hydrophilic coating layer on surfaces thereof were obtained and each evaluation was performed in the same manner as in Example 1 except that the hydrogel, the first polymer, and the second polymer were changed as shown in Table 3.

### <Example 3>

The hydrogel adjusted in Synthesis Example 1-3 was used as the hydrogel. The hydrogel was immersed in the first polymer solution adjusted in Synthesis Example 2-2, and heated under a condition of 90°C and 2 hours, thereby bonding the hydrogel and the first polymer to each other via a covalent bond and forming a first coating layer on a surface of the hydrogel.

Next, the hydrogel having the first coating layer was immersed in a physiological saline solution containing 0.1 mass% of the second polymer adjusted using the second polymer solution adjusted in Synthesis Example 3-2, and was subjected to a sterilization treatment under a condition of 121°C and 20 minutes, thereby obtaining a contact lens in which the second polymer was bonded to the first polymer via a covalent bond and a hydrophilic coating layer on the surface, and each evaluation was performed.

### <Comparative Example 1>

Methafilcon A (contact lens made of a copolymer of HEMA and MAA) was used as the hydrogel. The hydrogel was immersed in the second polymer solution adjusted in Synthesis Example 3-3, and heated at 75°C for 2 hours in the presence of triethylamine in an amount of catalyst, thereby obtaining a contact lens in which the second polymer was bonded to the hydrogel via a covalent bond and only the second polymer was coated on the surface of the hydrogel.

The obtained contact lens was sufficiently washed with water, then immersed in a physiological saline solution adjusted in accordance with ISO18369-3, and subjected to a sterilization treatment under a condition of 121°C and 20 minutes, and each evaluation was performed.

### <Comparative Example 2>

The hydrogel adjusted in Synthesis Example 1-1 was used. The hydrogel was immersed in the first polymer solution adjusted in Synthesis Example 2-1, and heated at 75°C for 2 hours in the presence of triethylamine in an amount of catalyst, thereby obtaining a contact lens in which the first polymer was bonded to the hydrogel via a covalent bond and only the first polymer was coated on the surface of the hydrogel.

The obtained contact lens was sufficiently washed with water, then immersed in a physiological saline solution adjusted in accordance with ISO18369-3, and subjected to a sterilization treatment under a condition of 121°C and 20 minutes, and each evaluation was performed.

### <Comparative Example 3>

The hydrogel adjusted in Synthesis Example 1-2 was used. The hydrogel was immersed in the second polymer solution adjusted in Synthesis Example 3-1 and heated at 75°C for 2 hours in the presence of triethylamine in an amount of catalyst. In this case, the hydrogel and the second polymer did not form a bond.

The obtained contact lens was sufficiently washed with water, then immersed in a physiological saline solution adjusted in accordance with ISO18369-3, and subjected to a sterilization treatment under a condition of 121°C and 20 minutes, and each evaluation was performed.

The contact lenses of Examples 1 to 4 had no difference in transparency, moisture content, elongation at break, and Dk as compared with the hydrogel before coating the first polymer and the second polymer. Further, the contact lenses of Examples 1 to 4 had good wettability and lubricity, which is different from that of the hydrogel before coating the first polymer and the second polymer.

In addition, the contact lenses of Examples 1 to 4 had good durability.

That is, since the surface of the contact lens has sufficient hydrophilicity and lubricity even in a case where the contact lenses of Examples 1 to 4 are continuously worn for a long period of time, a user can use the contact lenses for a long period of time without a feeling of discomfort.

In the contact lens of Comparative Example 1, only the second polymer was bonded to the hydrogel, and the wettability and the lubricity of the contact lens after the coating treatment were good, but since the lubricity was reduced after washing the contact lens for 14 times, and the wettability was also reduced after washing the contact lens for 30 times, the durability of the coating was not sufficient.

In the contact lens of Comparative Example 2, only the first polymer was bonded to the hydrogel, and the wettability and the lubricity of the contact lens after the treatment were good, but since the lubricity was reduced after washing the contact lens for 14 times, and the wettability was also reduced after washing the contact lens for 30 times, the durability of the coating was not sufficient.

Since the contact lens of Comparative Example 3 was not subjected to a coating treatment with the first polymer, the hydrogel and the second polymer could not be bonded, and the surface of the contact lens was not hydrophilized. Therefore, the contact lens after the coating treatment was inferior in wettability and lubricity.

**[Table 1]**

| | | Synthesis Example 1-1 | Synthesis Example 1-2 | Synthesis Example 1-3 |
|---|---|---|---|---|
| Glycidyl group-containing monomer | GdMA | 2.0 | 2.0 | 2.0 |
| Other monomers | HEMA (hydrophilic monomer) | - | 15.0 | 97.5 |
| | HBMA (hydrophilic monomer) | 12.5 | - | - |
| | NVP (hydrophilic monomer) | 30.0 | 17.5 | - |
| | DMAA (hydrophilic monomer) | - | 15.0 | - |
| | MMA (hydrophobic monomer) | 5.0 | - | - |
| | SiGMA (silicone-containing monomer) | 20.0 | - | - |
| | ETS (silicone-containing monomer) | - | 15.0 | - |
| | MCR-M11 (silicone-containing monomer) | 30.0 | 35.0 | - |
| | TEGDMA (crosslinking agent) | 0.5 | 0.5 | 0.5 |
| Subtotal | | 100.0 | 100.0 | 100.0 |
| Solvent | EtOH | - | - | 20.0 |
| | NPA | 100 | - | - |
| Initiator | AIBN | 0.5 | - | 0.5 |
| | I-819 | - | 0.5 | - |
| Total | | 110.5 | 100.5 | 120.5 |
| Polymerization method | | Thermal polymerization | Photopolymerization | Thermal polymerization |
| Polymerization condition | | 100°C 2 hours | 1.5 mW/cm² 30 minutes | 100°C 2 hours |

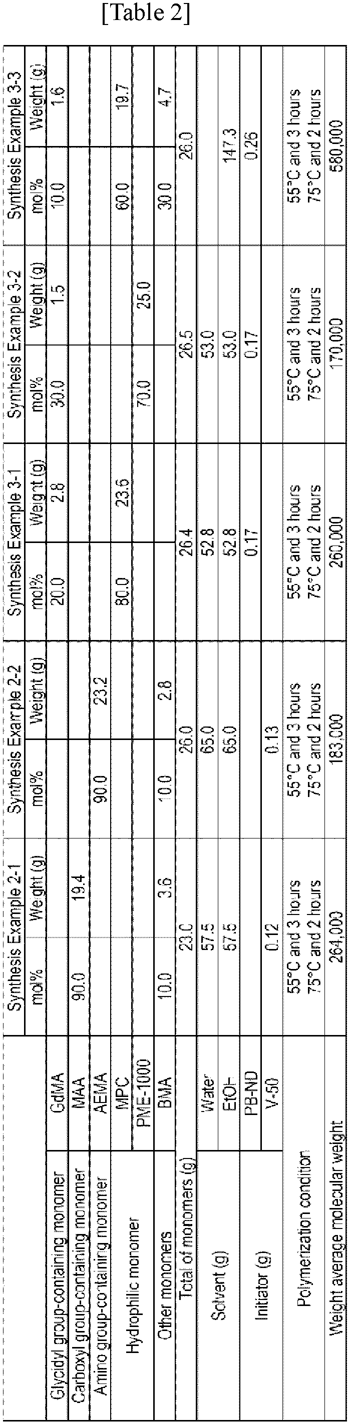

**[Table 3]**

| | | | Example 1 | Example 2 | Example 3 | Example 4 |
|---|---|---|---|---|---|---|
| Hydrogel | | | Synthesis Example 1-1 | Synthesis Example 1-2 | Synthesis Example 1-3 | Synthesis Example 1-2 |
| First polymer | | | Synthesis Example 2-1 | Synthesis Example 2-1 | Synthesis Example 2-2 | Synthesis Example 2-3 |
| Reactive group of first polymer | | | Carboxyl group | Carboxyl group | Amino group | Carboxyl group |
| Second polymer | | | Synthesis Example 3-1 | Synthesis Example 3-3 | Synthesis Example 3-2 | Synthesis Example 3-3 |
| Evaluation results of hydrogel before coating treatment of first and second polymers | Transparency | | Transparent | Transparent | Transparent | Transparent |
| | Moisture content (%) | | 48 | 41 | 38 | 41 |
| | Elongation at break (%) | | 200 | 230 | 250 | 230 |
| | Dk | | 100 | 130 | 10 | 130 |
| | WBUT (s) | | 3 | 1 | 5 | 1 |
| | Lubricity | | 4 | 2 | 2 | 2 |
| Evaluation results of contact lens after coating treatment of first and second polymers | Transparency | | Transparent | Transparent | Transparent | Transparent |
| | Moisture content (%) | | 48 | 41 | 38 | 41 |
| | Elongation at break (%) | | 210 | 230 | 230 | 230 |
| | Dk | | 100 | 130 | 10 | 130 |
| | WBUT (s) | | > 10 | > 10 | > 10 | > 10 |
| | Lubricity | | 9 | 7 | 8 | 9 |
| | Durability of coating | Washing for 14 times | A | A | A | A |
| | | Washing for 30 times | A | A | A | A |

**[Table 4]**

| | | | Comparative Example 1 | Comparative Example 2 | Comparative Example 3 |
|---|---|---|---|---|---|
| Hydrogel | | | Methafilcon A | Synthesis Example 1-1 | Synthesis Example 1-2 |
| First polymer | | | - | Synthesis Example 2-1 | |
| Functional group of first polymer | | | - | Carboxyl group | - |
| Second polymer | | | Synthesis Example 3-3 | - | Synthesis Example 3-1 |
| Evaluation results of hydrogel before treatment of first and second polymers | Transparency | | Transparent | Transparent | Transparent |
| | Moisture content (%) | | 58 | 48 | 41 |
| | Elongation at break (%) | | 250 | 200 | 230 |
| | Dk | | 15 | 100 | 130 |
| | WBUT (s) | | 8 | 3 | 1 |
| | Lubricity | | 3 | 4 | 2 |
| Evaluation results of contact lens after treatment of first and second polymers | Transparency | | Transparent | Transparent | Transparent |
| | Moisture content (%) | | 58 | 48 | 41 |
| | Elongation at break (%) | | 250 | 220 | 230 |
| | Dk | | 15 | 110 | 130 |
| | WBUT (s) | | > 10 | > 10 | 1 |
| | Lubricity | | 8 | 6 | 2 |
| | Durability of coating | Washing for 14 times | B | B | Not evaluated |
| | | Washing for 30 times | C | C | Not evaluated |

### INDUSTRIAL APPLICABILITY

An object of the present invention is to provide a soft contact lens having sufficient hydrophilicity and lubricity on a surface even in a case where the soft contact lens is worn continuously for a long period of time.

Although the present invention has been described in detail and with reference to specific embodiments, it will be apparent to those skilled in the art that various changes and modifications can be made without departing from the spirit and scope of the present invention.

The present application is based on a Japanese patent application (Japanese Patent Application No. 2022-090437) filed on June 2, 2022, the contents which are incorporated herein by reference.

## Claims

1. A soft contact lens comprising the following (1), (2) and (3):
(1) a hydrogel (P1) having a glycidyl group and containing a glycidyl (meth)acrylate constituent unit;
(2) a first coating layer formed on a surface of the hydrogel (P1) and having a carboxyl group and/or an amino group, the first coating layer comprising a first polymer (P2) containing a constituent unit derived from a carboxyl group-containing monomer and/or a constituent unit derived from an amino group-containing monomer, and/or polysaccharides having a carboxyl group and/or an amino group; and
(3) a second coating layer formed on a surface of the first coating layer and having a glycidyl group, the second coating layer comprising a second polymer (P3) containing a constituent unit derived from glycidyl (meth)acrylate, and a constituent unit derived from 2-(meth)acryloyloxyethyl phosphorylcholine, polyethylene glycol (meth)acrylate, and/or methoxypolyethylene glycol methacrylate, wherein
the glycidyl group of the hydrogel (P1) is bonded to the carboxyl group and/or the amino group of the first coating layer via a covalent bond, and the carboxyl group and/or the amino group of the first coating layer is bonded to the glycidyl group of the second coating layer via a covalent bond.

2. The soft contact lens according to claim 1, wherein
the first coating layer comprises a first polymer (P2) containing a constituent unit derived from (meth)acrylic acid and/or a constituent unit derived from 2-aminoethyl (meth)acrylate.

3. The soft contact lens according to claim 1, wherein
the second coating layer comprises a second polymer (P3) containing a constituent unit derived from 2-methacryloyloxyethyl phosphorylcholine.

4. A method of manufacturing the soft contact lens according to any one of claims 1 to 3, the method comprising the following steps:
(1) a step of immersing the hydrogel (P1) in a solution in which the first polymer (P2) and/or the polysaccharides having a carboxyl group and/or an amino group are dissolved; and
(2) a step of immersing the hydrogel having the first coating layer formed thereon after the immersion in (1) in a solution in which the second polymer (P3) is dissolved.
